# EUROPEAN PATENT APPLICATION

(11) **EP 1 847 265 A1**
(43) Date of publication of application: **24.10.2007**
(21) Application number: 05700483.0
(22) Date of filing: 26.01.2005
(51) Int. Cl.: A61K 31/352, A61P 13/12, A61P 19/08

(54) **THE USE OF FLAVONOL AND FLAVONOL GLYCOSIDES FOR STIMULATING GROWTH OF RENAL TUBULAR EPITHELIAL CELLS TO SECRETE THE KIDNEY SECRETING BONE GROWTH FACTOR**

(71) Applicant: Long, Mian, Honolulu, HI 96822 (US)
(72) Inventor: Long, Mian, Honolulu, HI 96822 (US)
(74) Representative: Lorente Berges, Ana
(86) International application number: PCT/CN2005/000110
(87) International publication number: WO 2006/079243

(57) **Abstract**

The present invention provides a method to diagnose the kidney function of recreating kidney secreted bone growth factor (KSBGF) by examining the blood concentration of KSBGF, a method to diagnose the state of bone formation by examining the blood concentration of KSBCF. a method to produce KSBGF, a method of using KSBGF to promote bone growth, a method for screening medicine for bone reformation, a method of using flavonol and flavonol glycosides to promote renal epithelial cell proliferation, and the application of flavonol and flavonol glycosides to improve renal function, to treat renal failure, and to promote bone formation.

## Description

### Field of the Invention:

The present invention is directed to a method for using flavonol and glycoside flavonol to promote renal tubular cell proliferation, a method to stimulate kidney secreting the kidney secreted bone growth factor (KSBGF), a process for using KSBGF to promote bone formation, a process to determine the quantity of KSBGF for diagnosing the kidney function of secreting KSBGF and to predict the state of bone formation, a process for screening for medicinal treatment designed to that promote bone formation, and the use of flavonol and glycoside flavonol for treating kidney deficiency and promoting bone formation.

### Background of the invention:

The role of the kidney in regulating bone formation can be seen in the patients with renal failure and associated secondary osteoporosis. In human and mammals, bones are continuously remodeled through repeated cycles of destruction and rebuilding. (Bone Health in the Balance. Paula Kiberstis, Orla Smith, and Colin Norman, Science 1 September 2000; 289: 1497 [DOI: 10.1126/ science.289.5484.1497]. Weak bone formation following by the renal failure has been seen in clinical studies, which is not recoverable by supplementing with vitamin D and calcium.

There is significant precedence for using herbal medicine for treating renal failure in Traditional Chinese Medicine. The theory that the "kidney controls the bone" was recorded in Traditional Chinese medicine. Practitioners observed that the kidney regulates bone metabolism, since the condition of patients with kidney deficiency was often accompanied by osteoporosis. Furthermore the herbal medicine that believed tonic for kidney promotes the bone formation in patients with fracture. But the active components present in the herbs and their principle effective components were unclear. It has not been observed in the literature that the kidney could secrete bone growth factor and in turn increase bone formation.
Bone Morphogenic Proteins (BMPs) are secreted proteins that play an important role in bone development and maturation. There are 15 BMP and two BMP receptors (BMPR I and BMPR II) which have been identified so far. (Bone morphogenetic proteins: relevance in spine surgery. Orthopedic Clinics of North America. 33(2):447-63, ix, Apr. 2002). Reddi et al. also reported that BMPs play an important role on the bone formation in healing fracture. (Bone morphogenetic proteins: from basic science to clinical applications, Journal of Bone & Joint Surgery - American Volume. 83-A Suppl 1(Pt 1): S1-6,2001). Lund et al.'s experiments showed the benefit of BMP7 on the osteoporosis associated with renal failure in animal models. (Lund RJ. Davies MR, Brown AJ, Hruska KA, Successful treatment of an adynamic bone disorder with bone morphogenetic protein-7 in a renal ablation model, Journal of the American Society of Nephrology, 15(2):359-69, Feb. 2004). Above examples confirmed the effects of BMP on bone metabolism. However the mechanism of production and secretion of BMPs are unclear.

### Summary of invention

One aspect of the present invention is directed to a process for promoting renal tubular cell proliferation that includes the process of applying flavonol and glycoside flavonol to stimulate tubular cell proliferation.

An additional aspect of the invention is directed to a method for producing KSBGF. In particular, it is to provide a process for renal tubular cell secreting KSBGF and/or a process for renal tubular cells get in touch with flavonol and flavonol glycoside.

An additional aspect of the invention is directed to a method for producing KSBGF. In particular, it is to provide a process for renal tubular cell secreting KSBGF and/or a process for renal tubular cells get in touch with flavonol and glycoside flavonol.

A further aspect of the invention is to provide a process for screening the medicine for treating the disease based on bone growth disorder. In particular, the invention is directed a process for the candidates get in touch with renal tubular cells and examine the producing of KSBGF.

A further aspect of the invention is to provide a process for using KSBGF for producing the medicine for improving bone formation. The KSBGF here does not include BMP.

In this invention, the flavonol includes one or more than one of the following flavonol: Datiscetin, Fisetin, Kaempferol, quercetin, tangeretin, nobiletin, azeleatin, 5-deoxykaempferol, galangin, glepidotin A, Gossypetin, herbacetin, 6-hydrykaempferol, isoquercitrin, isorhamnetin, kaempferide, morin, myricetin, patuletin, quercetagetin, rhamnetin, robinetin and Seangularetin. The advanced choice is fisetin, Kaempferol, quercetin, etc. The flavonol glucoside includes one or more than one of the follow flavonol: icariin, gossypin, rutin, hyperin, myricitrin, kaempferitrin, Fesetin 8-c-glucoside quercitrin, quercimeritrin and robinetin. The advanced choice is icariin.

The present invention is to provide an application of flavonol and glycoside flavonol in producing medicines for renal failure and promoting bone formation.

### Figure caption:

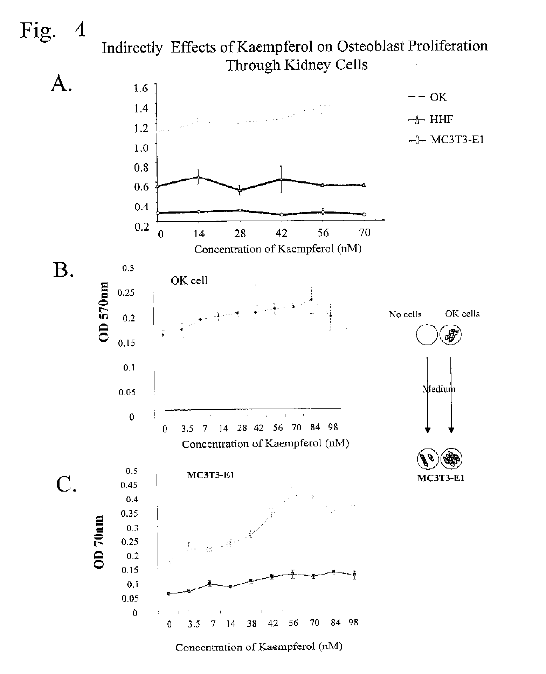

Fig 1. Shows the structure of flavonols, Glycoside flavonols and Apigenin, Isokaempferol (Apigenin and Isokaempferol are not Flavonol, but. Flavonoid. Flavonoid with a 3-OH is Flavonol, or with a 3-o-glycoside is glycoside flavonol).
Fig 2A shows that flavonols and glucoside flavonol increase opossum kidney (OK) cell proliferation and Apigenin did not increase OK cell proliferation. The higher numbers of OD, the greater cell growth.
Fig 2B Shows isokaempferol did not increase ok cell proliferation.
Fig 3A Shows the OK cell conditioned-medium (OKM) both with and without flavonol and glycoside flavonol increase osteoblast proliferation. Fig 3B shows Flavonol and flavonol glycoside, Apigenin and Isokaempferol do not directly increase osteoblast proliferation.
Fig 4A shows the effect of kaempferol at various concentrations on kidney cell, osteoblast and fibroblast cell proliferation.
Fig 4B shows the process of preparation of OKM and the kidney cell growth in various concentrations of kaempferol for 24 hours.
Fig C shows osteoblast growth in OKM treated with various concentrations of kaempferol (from Fig 4B). At the concentration of kaempferol at 0, the data show OKM significantly increased the osteoblast proliferation compared with the CM. Kaempferol conditioned-OKM increasing osteoblast proliferation with a dose response. All the dada together indicate that kidney cells secrete KSBGF, that in turn promotes bone formation. Kaempferol promotes kidney cell secreted KSBGF to improve bone formation.

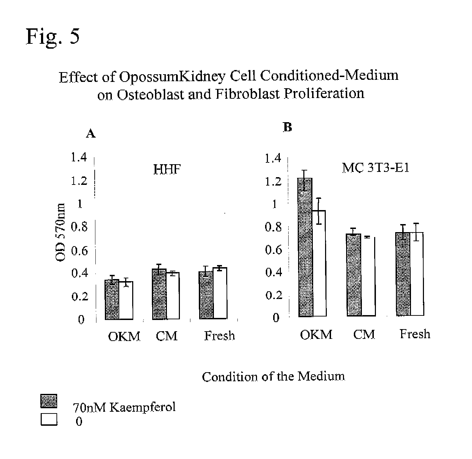

Fig 5A shows HFF cell growth in OKM, CM and fresh medium under the condition with and without kaempferol. HFF cell grow in OKM was similar to that in CM and fresh medium. OKM does not promote HFF cell proliferation.
Fig 5B: shows OKM without kaempferol significantly increased osteoblast cell proliferation. OKM with additional 70nM kaempferol stimulation significantly increased osteoblast cell proliferation. The CM and fresh medium both with and without kaempferol did not increase human fibroblast proliferation.

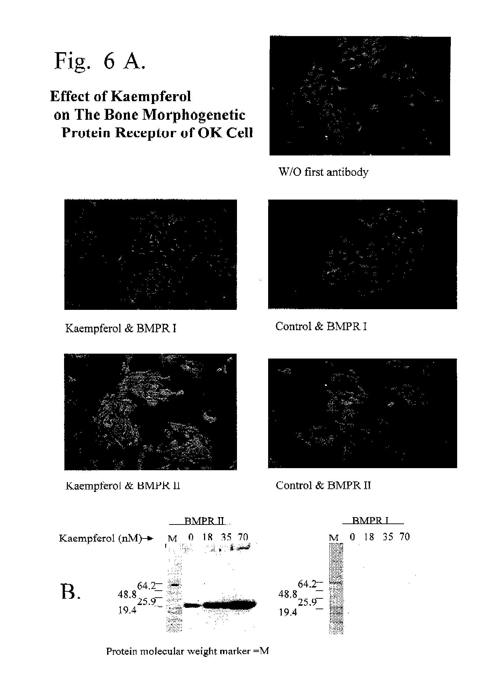

Fig 6A shows the immunostain by using anti-BMP receptor II and I on OK cell. Although BMPR I was not been found on OK cells, BMPR II was found on OK cells. Furthermore, the presence of kaempferol increased the density of BMPR II on OK cells. Fig 6B shows Western Blot of OK cell by using anti-BMPR I and II. Again BMPR I also was not found in OK cells sample, BMPR II was. Similarly, the presence of kaempferol in the medium increased BMPR II on OK cells.

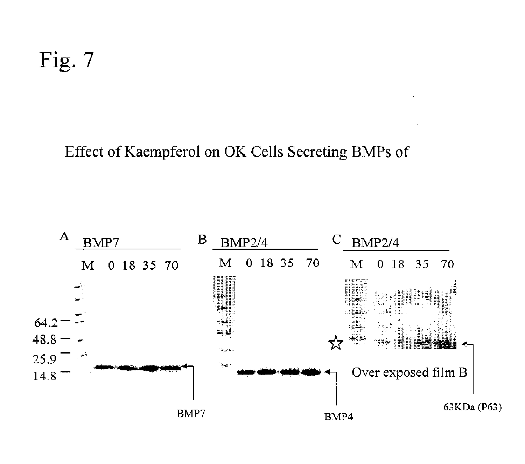

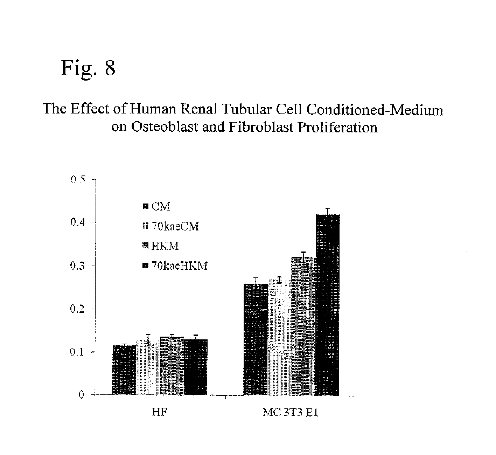

Fig 7 shows the quantity of BMP 2/4 and 7 in the kaempferol conditioned-OKM. BMP7 was increased in dose response to kaempferol concentration in the OK cell medium, as was BMP2/4. On the over exposed film reacted with anti-BMP 2/4 (Same as in Fig 7C), a protein with the molecular weight about 63KDa (P63) was found. This quantity of P63 also increased with the dose of kaempferol.

In this invention, the flavonol is designated that there is 3-OH on the flavones. The flavonol glucoside is designated that there is 3-o-aglycone or aglycones. In this invention, the flavonol are include, but not only include : datiscetin, fisetin, kaempferol, quercetin, tangeretin, nobiletin, azeleatin, 5-deoxykaempferol, galangin, glepidotin A, gossypetin, herbacetin, 6-hydrykaempferol, isoquercitrin, isorhamnetin, kaempteride, morin, myricetin, patuletin, quercetagetin, rhamnetin, robinetin and seangularetin. The advanced choice is fisetin, kaempferol, quercetin, etc. The flavonol glucosides include, but not only include: icariin, gossypin, rutin, hyperin, myricitrin, kaempferitrin, Fesetin 8-c-glucoside quercitrin, quercimeritrin and robinetin. The advanced choice is icariin.

If there is no a 3-OH on the flavone, such as apigenin, it is not flavonol. If there is no an aglucone, but other group, it is not flavonol glucoside, such as isokaempferol, which is not the flavonol includes in this invention.

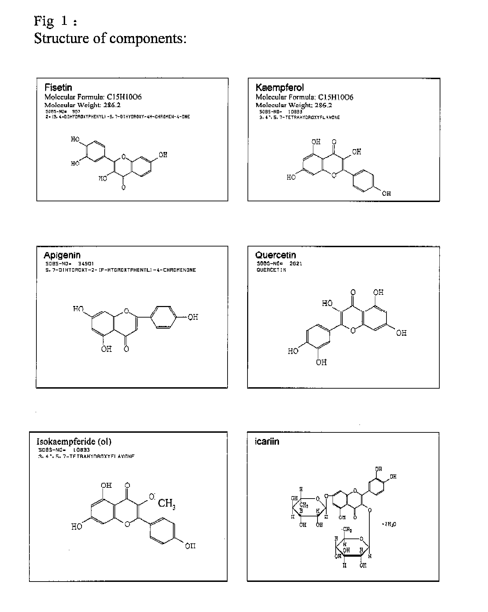

The structure of the advanced choice flavones were showed on the fig 1.

This applicant excitedly found that renal tubular cells secrete KSBGF. It implies that the kidney is the organ that secretes KSBGF. In addition, this applicant found that flavonol and glycoside flavonol stimulate renal tubular cells secreted significantly more KSBGF. This further confirms that the kidney is the organ that secretes KSBGF.

In this invention, the concepts of KSBGFs are the factors which are secreted from the kidney and which stimulates bone formation. These include but are not limited to BMP 2, 4, 7, 8, 9 and 10.

Based on practicing scheme, this invention is to provide a process for promoting renal tubular cell proliferation. It includes the above definition that the process of renal tubular cells get in touch with the flavonol and glycoside flavonol. More specifically, the appropriate condition of the medium for kidney cell growth is allowed to be choice.

Based on other practicing scheme, this invention is directed to a process for producing KSBGF, which includes the processing of renal tubular cell secreted KSBGF. As discussed above renal tubular cells secrete KSBGF, therefore, to culture renal tubular cells, which in the right condition with right medium may produce KSBGF. In this aspect, this invention includes the process of the renal tubular cells get in touch with flavonol and glycoside flavonol. This means adding flavonol or glycoside flavonol into culture medium to increase the secretion of KSBGF. Based on this invention, the KSBGF can been isolated and identified from the medium.

Based on another practicing scheme, this invention is directed to a process for using KSBGF for treating the disease of bone formation.

Based on another practicing scheme, this invention is directed toward a process for screening the medicine for promoting bone growth and treating bone growth diseases, Furthermore, it includes the process for using the candidates to get in touch with renal tubular cells, then examine the producing of KSBGF. In detail, culture the candidate in the possible condition of medium with renal tubular cells and then examine the concentration of KSBGF. The control sample will be the same condition but without zero testing sample. The active component will increase the KSBGF after incubation.

Based on practicing scheme, this invention is directed toward a method for treating renal failure. This includes administrate one or more than one the effective dose of flavonol or flavonol glycoside.

Based on another practicing scheme, this invention is directed toward a process for promoting bone formation and treating disease of bone formation, which includes administering the effective dose of Flavonol and glyciside Flavonol to patients. Based on this process, flavonol and glyciside Flavonol can be used for treating bone diseases such as osteoporosis.

The term "effective dose" indicates the dose of the medicine which effect on the individual. The exactly dose depends on the purpose of the treatment which may be determined by professionals in this field using the known method. It depends on the methods to administrate the medication, orally or externally, different location, patient's status, including age, body weight, sex, health status, dietary habit, time to administrate the medication, the interaction with other medication, and the severity of the disease. It also can be determined by professionals in this field using the conventional experiments.

The term "patient" here includes human and animal (especially the mammals) and other organism. Therefore, the medicine included in this invention can be useful fur human and animal. The advanced choice of the patient for using this medicine are mammals. The best advanced choice of patients for using this medicine are human.

The medication in this invention can be administrated in various ways: include, but not limited to, orally, subcutaneous, intravenous, intranasal, intracutaneous, intraperitoreal, intramusculal, intrapulmonary, intravaginal, intrarectal or intraocular.

The medical dosage form includes the composition in the invention or other salt or solution and one or more than one excipient. As known by the professors in the field of pharmacology that, medical excipiet is a complementary composition, that improve the transfer of the dosage form. The excipients including chemical such as inactive composition, synergist or components which may improve the medical effect. Such as the excipients may improving the property of flow, homogenization, stability of products, tasting and aspect, that simplify the manufactory process and administration of medicine. Therefore the excipient are in active, but the property and contents of the components in the invention absent some relationship.

### Example:

The examples listed below explained this invention. They are not limited to the area of this invention.
All of the culture medium used for the following experiments was from Gibco. (No vitamin D was contained)
Except when otherwise indicated, opossum kidney tubular cells were used as the kidney cell line. These cells were obtained from American Type Culture Collection (ATCC), Manassas, VA. Osteoblasts used MC 3T3 E1 cells from Technologies Grang Island, NY. The fibroblast used were human heart fibroblast from ATCC. All antibodies were from Santa Cruz Biotechnology Inc.

### Example 1

### Flavonol increased renal tubular cell proliferation

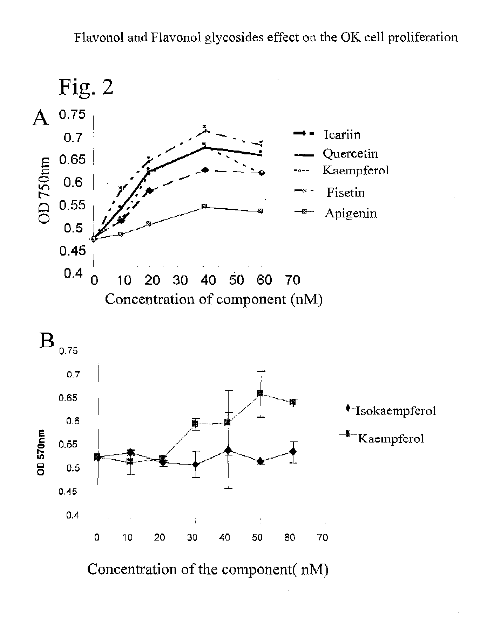

Ok cells at 1×10⁴/ml (0.1ml) were added into 96 wells plate. Several flavonol or the control flavonoid (which are not flavonol) were added to make the final concentration at 0-60nM.
After three days incubation, the cell growth was examined with CellTiter 96 at 570nM. The higher OD, the higher the numbers of living cells. (Fig 2.
Fig 2A shows Flavonol and glycoside flavonol increase kidney tubular cell proliferation, but non-flavonol component, apigenin and isokaempferol did not show the same effect.

The structure of apigenin and isokaempferol are similar to flavonol, but either the 3-OH was absent or blocked by a -CH3. This indicates that 3-OH or 3-0-glycoside on structure of flavonol is the functional group. on promoting renal tubular cell proliferation.
Therefore, this invention is directed toward the process of using flavonol for producing medicine for treating renal failure.

### Example 2

### Flavonol and glycoside flavonol do not directly stimulate osteoblast proliferation, but the OKM and the flavonol conditioned OKM promote osteoblast proliferation.

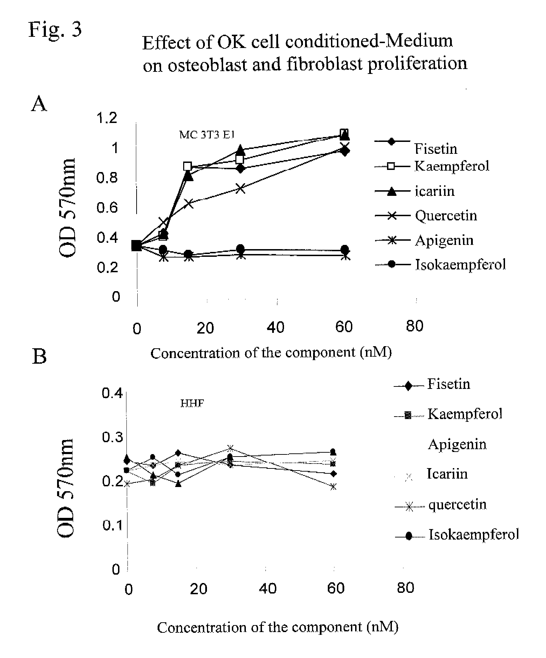

In this example, OK cells were used as the kidney tubular cell. OK cells at 6X10⁴ml (3ml) were added into 6 wells plate and various concentrations of flavonol, glycoside flavonol, apigenin and isokaempferol were applied. At the same time, the control culture medium (CM) with the same condition as above but without cells was prepared. After 24 hours incubation, the medium was collected for osteoblast culture. The OK cell growth was examined as discussed above.
In this example, the osteoblast was the MC 3T3 E1 cell. MC 3T3 E1 at 1×10⁵/ml (20µl) was added into 96 wells plate. Additionally, 80µL of OKM or CM were recruited. After 3 days incubation, the osteoblast growth was examined as discussed above. The results were showed on the Fig 3.
Fig 3 shows that MC 3T3 E1 cell growth in the OKM was significantly increased compared with those in the CM. MC 3T3 E1 cell growth in the flavonol conditioned-OKM was increased more than in the control OKM. MC 3T3 E1 growth in the non-flavonol conditioned-OKM was no increaser than in the control.

Fig 3B shows that there is promoting effect of flavonol on the MC 3T3 E1 cell growth.
The above data indicate that OKM stimulates osteoblast proliferation. However, the figure also demonstrated that the effect of flavonol conditioned-OKM on osteoblast proliferation was much greater than control OKM.
More specifically, based on the findings that renal tubular cells secrete KSBGF,this invention is directed toward a process to examine the concentration of KSBGF in body fluid for diagnosing kidney function in the secreting of KSBGF. Furthermore, the invention also is directed toward to examine the concentration of KSBGF in the body fluid for prognosticating the state of bone formation. Since renal tubular cells are able to produce KSBGF, the secretion of which can be promoted by flavinol and glycoside flavonol. It follows that flavonol and glycoside flavonol can therefore be used for making medicine for promoting bone formation.
In another aspect, the secreting of KSBGF from renal tubular cell was increased through stimulation by flavonol. Therefore, scientists may test the effect of the candidates on the renal tubular cell proliferation, or on the tubular cell secretion of KSBGF, or test the effect of the candidate conditioned-OKM on the osteoblast proliferation for screening medicine for promoting bone formation.

### Example 2

### Flavonol and glycoside flavonol do not directly stimulate osteoblast proliferation, but the OKM and the flavonol conditioned OKM promote osteoblast proliferation.

In this example, OK cells were used as the kidney tubular cell. OK cells at 6X10⁴ml (3ml) were added into 6 wells plate and various concentrations of flavonol, glycoside flavonol, apigenin and isokaempferol were applied. At the same time, the control culture medium (CM) with the same condition as above but without cells was prepared. After 24 hours incubation, the medium was collected for osteoblast culture. The OK cell growth was examined as discussed above.
In this example, the osteoblast was the MC 3T3 E1 cell. MC 3T3 E1 at 1 × 10⁵/ml (20µl) was added into 96 wells plate. Additionally, 80µL of OKM or CM were recruited. After 3 days incubation, the osteoblast growth was examined as discussed above. The results were showed on the Fig 3.
Fig 3 shows that MC 3T3 E1 cell growth in the OKM was significantly increased compared with those in the CM. MC 3T3 E1 cell growth in the flavonol conditioned-OKM was increased more than in the control OKM. MC 3T3 E1 growth in the non-flavonol conditioned-OKM was no increaser than in the control.
Fig 3B shows that there is promoting effect of flavonol on the MC 3T3E1 cell growth.
The above data indicate that OKM stimulates osteoblast proliferation. However, the figure also demonstrated that the effect of flavonol conditioned-OKM on osteoblast proliferation was much greater than control OKM.
More specifically, based on the findings that renal tubular cells secrete KSBGF,this invention is directed toward a process to examine the concentration of KSBGF in body fluid for diagnosing kidney function in the secreting of KSBGF. Furthermore, the invention also is directed toward to examine the concentration of KSBGF in the body fluid for prognosticating the state of bone formation Since renal tubular cells are able to produce KSRGF, the secretion of which can be promoted by flavinol and glycoside flavonol. It follows that flavonol and glycoside flavonol can therefore be used for making medicine for promoting bone formation.
In another aspect, the secreting of KSBGF from renal tubular cell was increased through stimulation by flavonol. Therefore, scientists may test the effect of the candidates on the renal tubular cell proliferation, or on the tubular cell secretion of KSBGF, or test the effect of the candidate conditioned-OKM on the osteoblast proliferation for screening medicine for promoting bone formation.

### Example 3.2

### Flavonol stimulates renal tubular cell secretion of KSBGF not only dependent on the increasing in kidney cell number.

Preparation of OKM and CM: OK cells at 2×10⁴/ml (0.1ml) mixed with various concentrations of kaempferol were incubated in 96 well plate. The medium made of the same condition as above without OK cells was also incubated in the 96 well plates. After 24 hour, the mediums were collected. The OK cell growth was examined.
MC 3T3 E1 cells at 1×10⁵/ml (20µl) were applied into 96 well plate along with OKM and CM (80µl). After 3 days incubation, the cell numbers were examined.
Fig 4Ashows OK cell growth in the mediun present with kaempferol was increase with dose response.
Fig 4B and 4C show MC 3T3 E1 cells growth in the both CM with and without kaempferol are significantly lower than in the OKM. MC 3T3 E1 cell growth in the kaempferol conditioned-OKM significantly increased proliferation compared with that in the control OKM. MC 3T3 E1 growth was not in direct ratio with the growth of OK cells.
In summary, the experiments indicate that OK cells secrete KSBGF which in turn promote MC 3T3 E1 cell proliferation (Fig 4C). Flavonols stimulate OK cells, which significantly increase the secretion of KSBGF. This effect increases with increased concentration of kaempferol. MC 3T3 E1 proliferation in the OKM was more than expected under the assumption that the increase is in direct ratio with the presented numbers of OK cells. It indicates that, flavonol and glycoside Flavonol promote osteoblast cell proliferation not only through increasing OK cell number, also increasing OK cell secrete KSBGF.
MC 3T3 E1 cell growth in both MC with or without kaempferol were similar. This indicates that the effect of Flavonol on increase of MC 3T3 cell growth was not directly. Rather it was dependent on the function of OK cells.
This experiment continued again that OK cells secrete KSBGF and flavonol increases the secretion.

### Example 4

### The effect of KSBGF on osteoblast proliferation is specially compared with fibroblast.

The preparation of OKM and CM: OK cells at 6×10⁴/ml (3ml) were incubated in 6 well plates over night. The medium with or without 70nM kaempferol was exchanged. The control medium (CM) was prepared under the same condition, without OK cells. After incubation for 24 hour, the medium was collected for MC 3T3 E1 and HFF cell growth experiments.
Effect of OKM on cell proliferation in MC 3T3 and HHF:
MC 3T3 E1 and HFF cell at 1X105/ml were added (20µl) into 96 well plates. In addition, 80µl of OKM, CM and fresh culture medium with or without kaempferol were applied. After 3 days incubation, the cell growth was examined and the results are shown in the fig 5A and 5B.
Fig 5A and 5B show that CM and fresh medium, with or without kaempferol, did not promote MC 3T3 E1 and HHF cell proliferation. OKM promoted MC 3T3 E1 cell proliferation, but did not promote HHF cell proliferation. Kaempferol conditioned-OKM additionally promoted MC 3T3 E1 cell proliferation, and also did not promote HHF cell proliferation.
This experiment indicates that compared with HFF, the stimulating effect of KSBGF was special on osteoblast cells.

### Example 5

### Flavonol and glycoside flavonol increase BMPR II on OK cell

(The detail of the experiment below is not translated. The method of this experiment are the standard. Please see the fig 6.)
The experiment shows BMPR I is absented on the OK cell. BMPR II is presented on OK cell. Flavonol increase the density of BMPR II on OK cell.
This experiment indicates that Flavonol and glycoside Flavonol increase BMPR II on OK cells. Therefore, it promotes kidney cell proliferation. Flavonol and glycoside Flavonol are able to be used for preparing medicine for promotion of kidney damage recovering and kidney function improving.

### Example 6: (It has not been repeated yet)

### Flavonol and glycoside flavonol stimulate OK cell secreting BMP 2/4, 7 and P63.

OKM was prepared in a 10cm plate with 6ml of OK cell (1×10⁴/ml). After incubation over nigh, the medium was removed and replaced with 3ml of FBS free medium. Kaempferol were added at the final concentration 0, 18, 35 or 70nM. After 24 hours incubation, the medium was collected. The medium was immune-precipitated with anti-BMP 2/4 or anti-BMP7. The precipitants were analyzed by western blot with anti-DMP2/4 or anti-BMP7 antibody.

### Immune-precipitate:

Anti BMP2/4 or 7 (20 µl) were added in to each 1 ml OKM and mixed well. It was set at 4°C for 60 min. Protein A -agars was added and mixed for 30 min. After centrifugation, the supernatant was removed and the bites were washed for three times. The sample solution was added and heated at 100°C for 5 min. After centrifugation the supernatant was removed and loaded to a 10% SDS gel.

After SDS gel was developed, the proteins were transfer to a polyvinylidene difluride membrane. After washed for three times, the membrane blocked with 5% skim milk and 0.02%xxxxx PBS for two hour, The membrane was reacted with 1:1,000anti-BMP 7 or 2/4. After reacted for two hour, the membrane was washed for 4 time with PBS. Then the membrane was reacted with 1: 3,000 second antibody for 1 hour. After stained, the picture was taken.

### Results:

Fig 7A shows that MBP7 was presented in the OKM. The quantity of BMP7 was increased by dose response of kaempferol. This indicates that OK cell secreted BMP7, and kaempferol increased the ability of OK cells to secrete BMP7.
Fig 7B shows that BMP2/4 was present in OKM. The quantity of BMP2/4 was increased by dose response of kaempferol. This indicates that OK cell secreted BMP2/4, and kaempferol increased the ability of OK cells to secrete BMP2/4.
Fig 7C shows 63kDa (P63) in the OKM. P63 was shown to be present after the film of 7B was over exposed. P63 was increased with dose response of kaempferol. According to the instruction from the manufacture, anti-BMP2/4 antibody may react with BMP8, 9 and 10 or other proteins. The P63 may be BMP 8, 9, 10 or another protein related to the BMP2/4.

Because the western blot of anti-BMP is a specific identification method, therefore, it indicated that KSBGF contains something other than BMPs.
Though the above examples were to illustrate this invention, the future improvements, corrections and changes are also included in this invention under the condition that they are not left out of the spirit and essence of this invention.

## Claims

1. A process for increasing renal tubular cell proliferation that includes the renal tubular cells get in touch with flavonol and the glycoside flavonol.

2. A process for producing KSBGF, that includes incubating renal tubular cell and utilizing them to secrete KSBGF.

3. The process of claim 2, wherein said renal tubular cells get in touch with flavonol or glycoside Flavonol.

4. The processes of claims 1-3, wherein said Flavonol includes one or more than one of the following flavonol: Datiscetin, Fisetin, Kaempferol, quercetin, tangeretin, nobiletin, azeleatin, 5-deoxykaempferol, galangin, glepidotin A, Gossypetin, herbacetin, 6-hydrykaempferol, isoquercitrin, isorhamnetin, kaempferide, morin, myricetin, patuletin, quercetagetin, rhamnetin, robinetin and Seangularetin.

5. The process of claim 4, wherein said flavonol included one or more than one of the follow flavonol: Fisetin, Kaempferol, quercetin.

6. The processes of claims 1-3, wherein said glycoside Flavonol includes icariin, rutin, kaempferitrin hyperin, myricitrin, Fesetin 8-c-glucoside quercitrin and robinetin.

7. The process of claim 6, wherein said glycoside flavonol is icariin.

8. The process of claim 2, wherein said KSBGF includes BMP.

9. The process of claim 2, wherein said KSBGF includes BMP 2,4,7,8,9,and/or 10.

10. The process of claim 2, wherein said KSBGF includes RMP 2, 4, 8, 9, and/or the molecular which are the antigen of BMP 2,4,7,8,9 and /or 10. or the molecule with molecular weight at about 63kDa.

11. The process of claim 2, wherein said process can be employed for examining the concentration of KSBGF in body fluids with the purpose of diagnosing kidney function in the secretion of KSBGF.

12. The process of claim 2, wherein said process examines the concentration of KSBGF for diagnosing the state of bone formation or the risk of osteoporosis.

13. The process of screening the medicine for treating disease of bone formation. It includes the process of the candidate components get in touch with renal tubular cells

14. The use of flavonol and glycoside flavonol in treating renal failure.

15. The use of flavonol and glycoside flavonol in promoting bone growth.

16. The processes of claims 14 or 15 wherein the said flavonol includes includes one or more than one of the following flavonol: Datiscetin, Fisetin, Kaempferol, quercetin, tangeretin, nobiletin, azeleatin, 5-deoxykaempferol, galangin, glepidotin A, Gossypetin, herbacetin, 6-hydrykaempferol, isoquercitrin, isorhamnetin, kaempferide, morin, myricetin, patuletin, quercetagetin, rhamnetin, robinetin and Seangularetin.

17. The processes of claim 14 or 15 wherein said glycoside flavonol includes fisetin, Kaempferol, quercetin

18. The processes of claim 14 or 15 wherein said glycoside flavonol includes icariin, rutin, kaempferitrin hyperin, myricitrin, Fesetin 8-c-glucoside quercitrin and robinetin.

19. The processes of claim 14 or 15 wherein said glycoside flavonol is icariin.

20. The process of KSBGF in promoting bone formation used in manufacturing medication.
